# EUROPÄISCHE PATENTANMELDUNG

(11) **EP 1 462 165 A1**
(43) Veröffentlichungstag der Anmeldung: **29.09.2004**
(21) Anmeldenummer: 04007403.1
(22) Anmeldetag: 26.03.2004
(51) Int. Cl.: B01J 29/70, B01J 29/86, B01J 29/90, B01J 37/08, C07C 209/60

(54) **Verfahren zur Herstellung von Alkylaminen und zur Erhöhung der Hydroaminierungsaktivität von calcinierten zeolithischen Katalysatoren**

(30) Priorität: 27.03.2003 DE 10313853
(71) Anmelder: BASF AKTIENGESELLSCHAFT, 67056 Ludwigshafen (DE)
(72) Erfinder: Sigl, Marcus, Dr., 68167 Mannheim (DE); Müller, Ulrich, Dr., 67435 Neustadt (DE)
(74) Vertreter: Isenbruck, Günter, Dr.

(57) **Zusammenfassung**

In einem Verfahren zur Herstellung von Alkylaminen durch Umsetzung von Olefinen mit Ammoniak, primären oder sekundären Aminen unter hydroaminierenden Bedingungen an einem calcinierten zeolithischen Katalysator wird der calcinierte zeolithische Katalysator maximal 24 Stunden vor Beginn der Umsetzung bei Temperaturen im Bereich von 100 bis 550°C in einem Gasstrom aus Luft, Stickstoff, anderen Inertgasen oder Gemischen davon thermisch aktiviert.

## Beschreibung

Die Erfindung betrifft ein Verfahren zur Herstellung von Alkylaminen durch Umsetzung von Olefinen mit Ammoniak, primären oder sekundären Aminen unter hydroaminierenden Bedingungen an einem calcinierten zeolithischen Katalysator. Ferner betrifft die Erfindung ein Verfahren zur Erhöhung der Hydroaminierungsaktivität von calcinierten zeolithischen Katalysatoren und die demgemäß erhaltenen Katalysatoren.

Die Verwendung von Zeolithen als Katalysatoren für die Aminierung von Olefinen mit Ammoniak oder primären oder sekundären Aminen ist seit längerer Zeit bekannt. So wird in der EP-A 0 133 938 ein Verfahren zur Herstellung von Aminen aus Olefinen und Ammoniak, primären, sekundären Aminen oder Gemischen derselben in Gegenwart eines Borosilikat- oder Borogermanatzeolithen vom Pentasil-Typ als Katalysator beschrieben. Die erhaltenen Amine werden aus dem Umsetzungsprodukt abgetrennt, und nicht umgesetzte Ausgangsstoffe werden zurückgeführt.

In der Anmeldung ist beschrieben, dass nach Deaktivierung der zeolithischen Katalysatoren durch Koksabscheidung während der Umsetzung die Katalysatoren durch Abbrennen der Koksablagerung mit Luft oder mit einem Luft/Stickstoff-Gemisch bei 400 bis 500°C, bevorzugt bei etwa 500°C, in einfacher Weise regeneriert werden können. Es ist beschrieben, dass die Katalysatoren dadurch ihre Anfangsaktivität zurückerhalten. Eine Aktivitätssteigerung von nicht derartig deaktivierten Katalysatoren ist nicht beschrieben.

EP-A 0 263 462 betrifft ebenfalls ein Verfahren zur Herstellung von Aminen aus Olefinen und Ammoniak, primären oder sekundären Aminen in Gegenwart eines chromhaltigen Borosilikat- oder eines chromhaltigen Eisensilikat-Zeolithen des Pentasil-Typs als Katalysator. Es wird beschrieben, dass calcinierte Eisen- und Borosilikatzeolithe mit Chrom dotiert werden können, indem sie mit einer Lösung einer Chromverbindung imprägniert werden. An die Imprägnierung schließt sich eine Trocknung an, auf die eine abermalige Calcinierung folgen kann. Ferner ist beschrieben, dass eine Nachbehandlung der dotierten Zeolithe mit Wasserstoff vorteilhaft sein kann. Ferner wird wiederum auf die Zurückerlangung der Anfangsaktivität von durch Koksabscheidung desaktivierten Katalysatoren hingewiesen.

Die WO 02/00597 betrifft ein Verfahren zur Herstellung von Alkylaminen, bei dem man in einer ersten Verfahrensstufe ein Olefin mit Ammoniak, einem primären Amin und/oder einem sekundären Amin unter hydroaminierenden Bedingungen umsetzt und anschließend das oder die erhaltenen Hydroaminierungsprodukte in einer zweiten Verfahrensstufe unter umalkylierenden Bedingungen umsetzt.

Es ist angegeben, dass insbesondere zeolithische Katalysatoren bei der Umsetzung eingesetzt werden können. Es wird des Weiteren angegeben, dass diese sauren Katalysatoren auch bereits gebrauchtes Material beinhalten oder aus solchem Material bestehen können, das durch übliche Methoden regeneriert wurde, beispielsweise durch eine Recalcinierung in Luft, Wasser, Kohlendioxid oder Inertgas bei Temperaturen > 200°C, durch Waschen mit Wasser, Säuren oder organischen Lösungsmitteln, durch Steamen oder durch Behandlung im Vakuum bei Temperaturen von mehr als 200°C.

Die WO 97/07088 betrifft ein Verfahren zur Herstellung von Aminen durch Umsetzung von Olefinen mit Ammoniak, primären oder sekundären Aminen in Gegenwart eines zeolithischen Katalysators. Dabei wird als zeolithischer Katalysator Bor-Beta-Zeolith eingesetzt. Es ist unter anderem beschrieben, dass die Bor-Beta-Zeolithe mit einem Bindemittel zu Strängen oder Tabletten verformt werden können. Nach der Verformung werden die Extrudate oder Presslinge getrocknet und calciniert, wobei die Calcinierung auch direkt im Aminierungsreaktor erfolgen kann. Ferner werden unterschiedliche Maßnahmen zur Erhöhung der Selektivität, der Standzeit und der Anzahl der möglichen Regenerierungen erwähnt, wobei auch auf Metallaufbringungen aus Lösungen eingegangen wird. In diesem Fall kann sich an eine Trocknung eine abermalige Calcination anschließen.

Eine Calcination im Reaktor bringt gegenüber einer Calcination außerhalb des Reaktors keinen Aktivitätsvorteil. Zudem ist der uncalcinierte Kontakt weich und würde deshalb beim Einfüllen in einen Reaktor, wie einen Rohrreaktor, zu Staub zerfallen. Die Härte erhalten die Strangkatalysatoren erst durch die Calcination.

Keine der Schriften beschreibt die Erhöhung der Hydroaminierungsaktivität von calcinierten zeolithischen Katalysatoren, die nicht durch eine Hydroaminierung deaktiviert wurden.

Aufgabe der vorliegenden Erfindung ist die Bereitstellung eines Verfahrens zur Erhöhung der Hydroaminierungsaktivität von calcinierten zeolithischen Katalysatoren, die nicht durch eine Hydroaminierung deaktiviert wurden, sowie eines entsprechenden Verfahrens zur Herstellung von Alkylaminen durch Umsetzung von Olefinen mit Ammoniak, primären oder sekundären Aminen unter hydroaminierenden Bedingungen an einem calcinierten zeolithischen Katalysator mit erhöhter Aktivität. Ferner soll ein entsprechender Katalysator mit erhöhter Aktivität bereitgestellt werden.

Die Aufgabe wird erfindungsgemäß gelöst durch ein Verfahren zur Herstellung von Alkylaminen durch Umsetzung von Olefinen mit Ammoniak, primären oder sekundären Aminen unter hydroaminierenden Bedingungen an einem calcinierten zeolithischen Katalysator, wobei der calcinierte zeolithische Katalysator maximal 24 Stunden vor Beginn der Umsetzung bei Temperaturen im Bereich von 100 bis 550°C in einem Gasstrom aus Luft, Stickstoff, anderen Inertgasen oder Gemischen davon thermisch aktiviert wird.

Die Aufgabe wird ferner gelöst durch ein Verfahren zur Erhöhung der Hydroaminierungsaktivität von calcinierten zeolithischen Katalysatoren, die nicht durch eine Hydroaminierung deaktiviert wurden, durch thermische Behandlung der calcinierten zeolithischen Katalysatoren bei Temperaturen im Bereich von 100 bis 550°C in einem Gasstrom aus Luft, Stickstoff, anderen Inertgasen oder Gemischen davon.

Es wurde erfindungsgemäß gefunden, dass durch eine thermische Aktivierung von zeolithischen Katalysatoren unmittelbar vor ihrer Verwendung deren Aktivität gesteigert werden kann. Dieser Effekt tritt insbesondere auch bei frischen Katalysatoren auf, die noch nicht in der Umsetzung eingesetzt wurden, sondern nach der Herstellung/Calcinierung für längere Zeiträume gelagert wurden. Es wurde beobachtet, dass zeolithische Katalysatoren nach ihrer Herstellung/Calcinierung innerhalb weniger Tage einen deutlichen Aktivitätsverlust erleiden. Das erfindungsgemäße Verfahren erlaubt es, nicht nur die Ausgangsaktivität der Katalysatoren, wie sie vor der Lagerung bestand, zu erreichen, sondern die Aktivität noch zu steigern.

Im Verfahren zur Erhöhung der Hydroaminierungsaktivität wird die thermische Behandlung vorzugsweise maximal 24 Stunden vor Beginn einer Hydroaminierung, in der der zeolithische Katalysator eingesetzt wird, durchgeführt. Im Verfahren zur Herstellung der Alkylamine wird ebenfalls der calcinierte zeolithische Katalysator maximal 24 Stunden vor Beginn der Umsetzung thermisch aktiviert.

Vorzugsweise wird der calcinierte zeolithische Katalysator maximal 6 Stunden, besonders bevorzugt maximal 2 Stunden, insbesondere unmittelbar vor Beginn der Umsetzung thermisch aktiviert. Der Ausdruck "maximal 24 Stunden vor Beginn der Umsetzung" bezieht sich auf den Zeitabstand zwischen dem Ende der thermischen Aktivierung und dem Beginn der Umsetzung in der Hydroaminierung. Im besonders bevorzugten Fall schließt sich an die thermische Aktivierung unmittelbar die Umsetzung an. Dies ist insbesondere vorteilhaft möglich, wenn die thermische Behandlung bereits im Aminierungsreaktor durchgeführt wird. In diesem Fall muss der Katalysator nach der thermischen Aktivierung nicht erst abgekühlt und in einen Aminierungsreaktor überführt werden, sondern kann direkt im Aminierungsreaktor verbleiben. Der zur thermischen Aktivierung eingesetzte Gasstrom aus Luft, Stickstoff, anderen Inertgasen oder Gemischen davon kann durch Ströme von Ammoniak, primären oder sekundären Aminen sowie von Olefinen ersetzt werden, so dass sich an die thermische Aktivierung die Umsetzung in der Hydroaminierung unmittelbar anschließt. Ein kurzzeitiges Abkühlen des Katalysators ist jedoch auch unkritisch. Je geringer der zeitliche Abstand zwischen thermischer Aktivierung und Beginn der Umsetzung ist, um so größer ist die Aktivität des calcinierten zeolithischen Katalysators.

Die thermische Aktivierung wird bei Temperaturen im Bereich von 100 bis 550°C, vorzugsweise 150 bis 525°C, insbesondere 200 bis 500°C durchgeführt. Dabei wird die thermische Aktivierung in einem Gasstrom aus Luft, Stickstoff, anderen Inertgasen oder Gemischen davon durchgeführt. Vorzugsweise werden Luft, Stickstoff oder Argon, insbesondere Luft, Stickstoff oder Luft/Stickstoff-Gemische eingesetzt. Üblicherweise wird unter Systemdruck bzw. Umgebungsdruck gearbeitet. Es kann jedoch auch unter vermindertem oder erhöhtem Druck gearbeitet werden. Geeignete Druckbereiche sind 0,5 bis 100 bar, besonders bevorzugt 1 bis 50 bar.

Die thermische Aktivierung wird dabei vorzugsweise für einen Zeitraum von 3 bis 50 Stunden, besonders bevorzugt 5 bis 40 Stunden, insbesondere 10 bis 25 Stunden durchgeführt. Der Zeitraum kann dabei je nach Temperatur und Lagerungszustand des zeolithischen Katalysators und nach den jeweiligen praktischen Erfordernissen ausgewählt werden.

Die thermische Behandlung kann außerhalb des Aminierungsreaktors erfolgen, beispielsweise in einem Drehrohr, Bandcalcinierer oder Hordenofen. Bevorzugt wird jedoch die Aktivierung direkt im Aminierungsreaktor durchgeführt. Dazu wird anstatt des Olefin/Ammoniak-Gemisches der vorgeheizte Gasstrom durch das Katalysatorbett geleitet. Der Katalysator liegt dabei insbesondere als Festbett vor.

Die erfindungsgemäße thermische Aktivierung wird dabei von der eigentlichen Calcinierung des zeolithischen Katalysators unterschieden. Die eigentliche Calcinierung wird vorzugsweise in einem Drehrohr durchgeführt, da eine für die Calcinierung notwendige Temperatur im Bereich von 400 bis 560°C im Drehrohr wesentlich besser zu realisieren ist als in einem Rohrreaktor. Rohrreaktoren sind üblicherweise für Reaktionstemperaturen zwischen 230 und 320°C ausgelegt. Die bei der Calcinierung entstehenden Nebenprodukte wie Wasser, Kohlenmonoxid, Kohlendioxid sowie Stickoxide stören zudem eine nachfolgende Aminierungsreaktion und müssten aus einem Aminierungsreaktor aufwendig entfernt werden.

In dem erfindungsgemäßen Verfahren werden calcinierte zeolithische Katalysatoren eingesetzt. Dies bedeutet, dass die Aktivmasse der Katalysatoren aus Zeolithen aufgebaut ist. Üblicherweise enthalten zeolithische Katalysatoren noch Bindemittel, die zur Herstellung von Katalysatorformkörpern erforderlich sind. Bei der Herstellung der Katalysatorformkörper aus entsprechenden Formmassen wird üblicherweise nach einer Trocknung noch calciniert, um zum letztendlichen Katalysator zu gelangen.

Der die Verformung der Katalysatorformkörper abschließende Schritt ist die Calcinierung. Hierbei wird in der Regel eine Temperatur von mehr als 400°C benötigt, damit das Bindermaterial aushärtet. Die Maximaltemperatur ist beschränkt durch die Stabilität des Zeolithen, der bei Temperaturen von mehr als 550°C seine Kristallinität verliert. Die Calcinierung wird großtechnisch im Drehrohr durchgeführt bei einer Temperatur im Bereich von 400 bis 560°C, einer Verweilzeit von 2 bis 4 Stunden. Im Labor wird üblicherweise in einem Ofen gearbeitet bei einer Temperatur von 480 bis 520°C und einem Zeitraum von 2 bis 32 Stunden.

Geeignete Katalysatoren für die Hydroaminierung von Olefinen mit Ammoniak und/oder einem primären Amin sind Zeolithe, insbesondere Faujasite wie X-, Y- und USY-Zeolith, Erionit, Chabazit, Mordenit, Offretit, Clinoptiolith, Pentasile wie ZSM-5 und ZBM-10, ZSM-11, ZSM-12, MCM-22, MCM-41, MCM-48, MCM-49, MCM-56, EMT, SSZ-26, SSZ-33, SSZ-37, CIT-1, PSH-3, NU-85, Beta sowie die borhaltigen Formen, wie zum Beispiel ZBM-11, H-Bor-ZSM-5, H-Bor-Beta, H-Bor-ZSM-11 sowie die gallium- oder titanhaltigen Formen. Sie zeichnen sich durch eine hohe Anzahl an katalytisch aktiven Zentren aus, kombiniert mit einer großen Oberfläche.

Die beschriebenen Zeolithe unterscheiden sich im Typ und in der Art der Nachbehandlung nach ihrer Herstellung (zum Beispiel thermische Behandlung, Dealuminierung, Säurebehandlung, Metallioneneintausch, etc.).

Beispiele für geeignete Zeolithe finden sich in US-A 4,375,002, US-A 4,536,602, EP-A 305 564, EP-A 101 921 und DE-A 42 06 992.

Auch die aus EP-A 133 938, EP-A 431 451 und EP-A 132 736 bekannten Zeolithe, bei denen es sich um Bor-, Gallium-, Alumino- und Eisensilikatzeolithe handelt, die gegebenenfalls wie beschrieben mit Alkali-, Erdalkali- und Übergangsmetallen dotiert werden können, sind geeignet.

Weiterhin sind zum Beispiel auch die aus CA-A 2 092 964 bekannten Beta-Zeolithe, die als kristalline Aluminosilikate bestimmter Zusammensetzung mit einer Porengröße von mehr als 5 Å definiert sind, geeignet.

Bevorzugt werden metall- oder halogenmodifizierte Beta-Zeolithe eingesetzt, wie zum Beispiel in DE-A 195 30 177 beschrieben.

Insbesondere geeignet sind auch Zeolith-Katalysatoren des Pentasil-Typs mit einem SiO₂/Al₂O₃-Molverhältnis von größer/gleich 10, wie sie in EP-A 132 736 offenbart sind.

Unter die Aluminiumphosphate und Silico-Alumophosphate fallen die kristallinen Systeme mit Zeolithstrukturen oder zeolithähnlichen Strukturen wie zum Beispiel SAPO-37, AlPO₄-5, SAPO-5, wie in DE-A 196 01 409 beschrieben, aber auch amorphe Systeme, wie sie zum Beispiel in DE-A 44 31 093 beschrieben sind. Sie besitzen allgemein die Formel Al₂O₃∗P₂O₅∗xSiO₂.

Die Katalysatoren können in Form von Pulver oder bevorzugt in Form von Formkörpern wie Strängen, Tabletten oder Splitt eingesetzt werden. Für die Verformung können 2 bis 60 Gew.-% (bezogen auf die zu verformende Masse) Bindemittel zugesetzt werden. Als Bindemittel eignen sich verschiedene Aluminiumoxide, bevorzugt Boehmit, amorphe Aluminosilikate mit einem molaren SiO₂/Al₂O₃-Verhältnis von 25:75 bis 95:5, Siliciumdioxid, bevorzugt hochdisperses SiO₂ wie zum Beispiel Silikasole, Gemische aus hochdispersem SiO₂ und hochdispersem Al₂O₃, hochdisperses TiO₂ sowie Tone.

Nach der Verformung werden die Extrudate oder Presslinge zweckmäßig bei 80 bis 150°C für 2 bis 16 Stunden, zum Beispiel bei 110°C/16 Stunden getrocknet und bei 300 bis 500°C/ 2 bis 16 Stunden calciniert, wobei die Calcinierung wie die Aktivierung auch direkt im Hydroaminierungsreaktor erfolgen kann.

In der Regel werden die Katalysatoren in der H-Form eingesetzt. Zur Erhöhung der Selektivität, der Standzeit und der Anzahl der möglichen Katalysatorregenerierungen kann man jedoch zudem verschiedene Modifizierungen an den Katalysatoren vornehmen.

Eine Modifizierung der Katalysatoren besteht darin, das man die unverformten Katalysatoren mit Alkalimetallen wie Na und K, Erdalkalimetallen wie Ca, Mg, Schwermetallen wie Tl, Übergangsmetallen wie beispielsweise Mn, Fe, Mo, Cu, Zn, Cr, Edelmetallen und/oder seltenen Erdmetallen wie zum Beispiel La, Ce oder Y ionenaustauschen bzw. dotieren kann.

Eine vorteilhafte Katalysatorausführungsform besteht darin, dass man die verformten Katalysatoren in einem Strömungsrohr vorlegt und bei 20 bis 100°C zum Beispiel ein Halogenid, ein Acetat, ein Oxalat, ein Citrat oder ein Nitrat der oben beschriebenen Metalle in gelöster Form darüberleitet. Ein derartiger Ionenaustausch kann zum Beispiel an der Wasserstoff-, Ammonium- und Alkaliform der Katalysatoren vorgenommen werden.

Eine andere Möglichkeit der Metallaufbringung auf die Katalysatoren besteht darin, dass man das zeolithische Material zum Beispiel mit einem Halogenid, Acetat, Oxalat, Citrat, Nitrat oder Oxid der oben beschriebenen Metalle in wässriger oder alkoholischer Lösung imprägniert.

Sowohl an einen Ionenaustausch als auch an eine Imprägnierung kann man eine Trocknung, wahlweise eine abermalige Calcination anschließen. Bei den metalldotierten Katalysatoren kann eine Nachbehandlung mit Wasserstoff und/oder mit Wasserdampf günstig sein.

Eine weitere Möglichkeit der Modifizierung des Katalysators besteht darin, dass man das heterogenkatalytische Material - verformt oder unverformt - einer Behandlung mit Säuren, wie Salzsäure (HCl), Flusssäure (HF), Phosphorsäure (H₃PO₄), Schwefelsäure (H₂SO₄), Oxalsäure (HO₂C-CO₂H) oder deren Gemischen unterwirft.

Eine besondere Ausführungsform besteht darin, dass man das Katalysatorpulver vor seiner Verformung mit Flusssäure (0,001 bis 2 molar, bevorzugt 0,05 bis 0,5 molar) 1 bis 3 Stunden unter Rückfluss behandelt. Nach Abfiltrieren und Auswaschen wird in der Regel bei 100 bis 160°C getrocknet und bei 400 bis 550°C calciniert.

Eine weitere besondere Ausführungsform liegt in einer HCl-Behandlung der Heterogenkatalysatoren nach ihrer Verformung mit Bindemittel. Hierbei wird der Heterogenkatalysator in der Regel 1 bis 3 Stunden bei Temperaturen zwischen 60 und 80°C mit einer 3 bis 25%igen, insbesondere mit einer 12 bis 20%igen Salzsäure behandelt, anschließend ausgewaschen, bei 100 bis 160°C getrocknet und bei 400 bis 550°C calciniert.

Eine andere Möglichkeit der Modifizierung des Katalysators ist der Austausch mit Ammoniumsalzen, zum Beispiel mit NH₄Cl, oder mit Mono-, Di- oder Polyaminen. Hierbei wird der mit Bindemittel verformte Heterogenkatalysator in der Regel bei 60 bis 80°C mit 10 bis 25%iger, bevorzugt ca. 20%iger NH₄Cl-Lösung 2 Stunden kontinuierlich in gewichtsmäßiger Heterogenkatalysator/Ammoniumchlorid-Lösung von 1:15 ausgetauscht und danach bei 100 bis 120°C getrocknet.

Eine weitere Modifikation, die an aluminiumhaltigen Katalysatoren vorgenommen werden kann, ist eine Dealuminierung, bei der ein Teil der Aluminiumatome durch Silicium ersetzt wird oder die Katalysatoren durch beispielsweise hydrothermale Behandlung in ihrem Aluminiumgehalt abgereichert werden. An eine hydrothermale Dealuminierung schließt sich vorteilhafterweise eine Extraktion mit Säuren oder Komplexbildnern an, um gebildetes Nichtgitteraluminium zu entfernen. Der Ersatz von Aluminium durch Silicium kann beispielsweise mit Hilfe von (NH₄)₂SiF₆ oder SiCl₄ erfolgen. Beispiele für Dealuminierungen von Y-Zeolithen finden sich in Corma et al., Stud. Surf. Sci. Catal. 37 (1987), Seiten 495 bis 503.

Die Katalysatoren kann man als Stränge mit Durchmessern von zum Beispiel 1 bis 4 mm oder als Tabletten mit zum Beispiel 3 bis 5 mm Durchmesser für die Hydroaminierung der Olefine einsetzen.

Die Umsetzung des Olefins mit Ammoniak und/oder dem primären Amin in Gegenwart der anorganischen Festkörpersäure kann zum Beispiel wie in EP-A 132 736, EP-A 752 409 und EP-A 822 179 beschrieben erfolgen.

Man geht dabei in der Regel so vor, dass man Ammoniak und/oder primäres Amin oder gegebenenfalls sekundäres Amin zusammen mit Olefin in einem molaren Verhältnis von 1:1 bis 10:1, bevorzugt 1:1 bis 5:1, mischt und in einem Festbettreaktor oder in einer Wirbelschicht bei einem Druck von 40 bis 700 bar, bevorzugt 200 bis 300 bar, und einer Temperatur von 80 bis 400°C, bevorzugt 230 bis 320°C, in der Gasphase oder im überkritischen Zustand umsetzt.

Alternativ kann die Reaktion in der Flüssigphase bei einem Druck von 40 bis 80 bar und einer Temperatur von 60 bis 120°C in einem Rührkessel, einem Fest-Flüssig-Fließbett oder einem Strömungsrohr durchgeführt werden.

Eine Ausführungsform dieses Verfahrens besteht darin, dass man Ammoniak und/oder das primäre bzw. sekundäre Amin zusammen mit dem Olefin oder dem Olefingemisch im molaren Verhältnis 1:1 bis 5:1 gemischt einem Festbettreaktor, der den zeolithischen Katalysator enthält, zuführt und bei einem Druck von 100 bis 320 bar, bevorzugt 150 bis 310 bar, insbesondere 200 bis 300 bar, und einer Temperatur von 200 bis 350°C, bevorzugt 220 bis 330°C, insbesondere 230 bis 320°C, in der Gasphase oder im überkritischen Zustand umsetzt.

Die Lage des Gleichgewichts und damit der Umsatz zum gewünschten Hydroaminierungsprodukt ist stark vom gewählten Reaktionsdruck abhängig. Hoher Druck begünstigt das Additionsprodukt, doch stellt im Allgemeinen aus technischen und wirtschaftlichen Gründen der Druckbereich bis 300 bar das Optimum dar. Die Selektivität der Reaktion wird - neben Größen wie Ammoniak-/Amin-Überschuss und Katalysator - in hohem Maß durch die Temperatur beeinflusst. Zwar nimmt die Reaktionsgeschwindigkeit der Additionsreaktion mit steigender Temperatur stark zu, doch werden gegebenenfalls selektivitätsmindemde Nebenreaktionen gleichzeitig gefördert. Zudem ist eine Temperaturerhöhung aus thermodynamischer Sicht meist nicht vorteilhaft. Die Lage des Temperaturoptimums bezüglich Umsatz und Selektivität ist von der Konstitution des Olefins, des eingesetzten primären Amins und des Katalysators abhängig und liegt meist im Bereich von 220 bis 320°C.

Im erfindungsgemäßen Verfahren werden Ammoniak, primäre oder sekundäre Amine eingesetzt. Die primären oder sekundären Amine weisen dabei vorzugsweise C₁₋₂₀-Alkylreste, besonders bevorzugt C₁₋₆-Alkylreste, insbesondere Methylreste oder Ethylreste auf.

Als Olefine können vorzugsweise C₂₋₂₀-Olefine eingesetzt werden, die aliphatisch sind. Sie können dabei linear oder verzweigt sein. Vorzugsweise werden C₂₋₁₂-Olefine, insbesondere C₂₋₆-Olefine eingesetzt. Beispiele geeigneter Olefine sind Ethen, Propen, Buten, Isobuten wie auch 1,3-Butadien.

Neben Ammoniak sind ganz besonders bevorzugte Amine Monomethylamin, Dimethylamin, Monoethylamin, Diethylamin, n-Butylamin, Isopropylamin, Diisopropylamin und Di-n-butylamin.

Hydroaminierungsprodukte ausgehend von Ethen und Ammoniak sind Mono-, Di- und/oder Triethylamin, ausgehend von Ethen und Monoethylamin: Di- und/oder Triethylamin, ausgehend von Isobuten und Ammoniak: tert.-Butylamin, ausgehend von 1,3-Butadien und Ammoniak: 1-Amino-3-buten und/oder 2-Amino-3-buten, ausgehend von 1,3-Butadien und n-Butylamin: (2-Butenyl)-n-butylamin und/oder (3-Butenyl)-n-butylamin und ausgehend von Propen und Isopropylamin: Diisopropylamin.

Die Umsetzung des Olefins mit dem Ammoniak und/oder dem Amin kann wie in der vorstehend genannten Literatur beschrieben erfolgen. Die Reaktion kann kontinuierlich, in Batch-Fahrweise oder in Semi-Batch-Fahrweise betrieben werden.

Bei den Batch-Fahrweisen wird der Katalysator vorzugsweise zusammen mit dem Amin vorgelegt. Nach Erreichen der Reaktionstemperatur wird das Olefin aufgepresst. Nach Absinken des Drucks wird das Produkt bzw. Produktgemisch abdestilliert. Überschüssiges Olefin sowie nicht abreagiertes Amin können zurückgeführt werden.

Bei der Batch-Fahrweise kann der Katalysator zusammen mit dem Produktgemisch über Sumpf aus dem Reaktor gefahren werden und separat aufgearbeitet werden. Die Reaktion kann in einem Rührkessel durchgeführt werden.

Geeignete diskontinuierliche und kontinuierliche Verfahren sind in WO 02/00597 sowie in der eingangs zitierten Literatur beschrieben. Besonders bevorzugt werden die Zeolithe als Formkörper eingesetzt, wobei die Umsetzung des Olefins mit Ammoniak bei Temperaturen von 200 bis 300°C und Drücken von 200 bis 300 bar erfolgt.

Die nach dem erfindungsgemäßen Verfahren zur Erhöhung der Hydroaminierungsaktivität von calcinierten zeolithischen Katalysatoren erhaltenen Katalysatoren weisen gegenüber nicht aktivierten Katalysatoren eine erhöhte Aktivität auf, die zu einer erhöhten Produktausbeute führt. Die Erfindung betrifft damit auch die nach den Aktivierungsverfahren erhältlichen Katalysatoren.

Vorzugsweise sind die im erfindungsgemäßen Verfahren eingesetzten Katalysatoren vor der thermischen Aktivierung nicht in einer Hydroaminierung eingesetzt worden. Es handelt sich damit um neue Katalysatoren, die vor der thermischen Aktivierung nur gelagert wurden, nicht jedoch in einer Umsetzung vorlagen. Hierdurch unterscheiden sich die im erfindungsgemäßen Verfahren eingesetzten Katalysatoren insbesondere von den Katalysatoren, die gemäß dem Stand der Technik recalciniert werden.

Die Erfindung wird durch die nachstehenden Beispiele näher erläutert.

### Beispiele:

Die Beispiele beziehen sich auf die Synthese von tert.-Butylamin (TBA) aus Isobuten und Ammoniak an Bor-Beta-Zeolith Kontakten. Die Herstellung des Bor-Beta-Zeolithen und dessen Verformung erfolgte analog den Angaben in EP 844 991 mit Boehmit als Bindemittel. Die Bindermenge wurde so gewählt, dass der fertig calcinierte Kontakt zu 26% aus Al₂O₃ besteht.

Die Performance-Untersuchungen wurden in einer kontinuierlich betriebenen Technikumsanlage durchgeführt. 10 g Katalysator als Splitt (1 - 1,6 mm) werden in einem Rohrreaktor (Innendurchmesser: 8 mm) mit einem Isobuten/Ammoniakgemisch (molares Verhältnis 1:1,5) durchströmt. Die Temperatur beträgt 270°C, der Druck 280 bar und die Belastung 2,6 g (Isobuten)/g(Kat.)h.

### Beispiel 1: (ex situ Regeneration)

Von einer frisch präparierten Charge Bor-Beta-Kontakt wurden nach unterschiedlichen Zeiten ( Tag, 6 Tage, 51 Tage) Proben gezogen und in der Technikumsanlage untersucht. Die restliche Menge wurde nach 58 Tagen im Ofen 16 Stunden unter Luftzufuhr bei 500°C aktiviert und sofort nach dem Abkühlen in den Reaktor eingebaut. Man erkennt, dass mit zunehmender Lagerung die TBA-Ausbeute (tert.-Butylamin) abnimmt, der nachcalcinierte Kontakt jedoch die Aktivität des nur einen Tag alten Kontakts sogar noch übertrifft.

| Lagerung | TBA-Ausbeute |
|---|---|
| 1 d | 15,0 mol% |
| 6 d | 13,9 mol% |
| 51 d | 13,2 mol% |
| 58 d, vor dem Einbau nachcalciniert | 15,5 mol% |

### Beispiel 2: (ex situ Regeneration)

Eine frisch präparierte Charge Bor-Beta-Kontakt wurde 3 Tage gelagert und dann in der Technikumsanlage untersucht. Aus derselben Charge wurde nach 21 Tagen erneut Kontakt eingebaut, vor dem Start der Reaktion jedoch 22 Stunden mit Stickstoff bei 300°C durchströmt. Der derart aktivierte Kontakt zeigt eine deutlich höhere TBA-Ausbeute als der unbehandelte Kontakt.

| Lagerung | TBA-Ausbeute |
|---|---|
| 3 d | 14,3 mol% |
| 21 d, im Reaktor mit N2 300°C gespült | 17,2 mol% |

### Beispiel 3: (in situ Regeneration)

Ein gelagerter Katalysator wurde in den Reaktor der Technikumsanlage eingebaut und zeigt eine Aktivität von 15,4 mol% TBA. Daraufhin wurde die Feedversorgung abgestellt und der Kontakt im Reaktor 22 Stunden bei 300°C mit Stickstoff durchströmt. Beim erneuten Anfahren wurde eine Aktivitätserhöhung auf 16,2 mol% TBA festgestellt.

## Patentansprüche

1. Verfahren zur Herstellung von Alkylaminen durch Umsetzung von Olefinen mit Ammoniak, primären oder sekundären Aminen unter hydroaminierenden Bedingungen an einem calcinierten zeolithischen Katalysator, **dadurch gekennzeichnet, dass** der calcinierte zeolithische Katalysator maximal 24 Stunden vor Beginn der Umsetzung bei Temperaturen im Bereich von 100 bis 550°C in einem Gasstrom aus Luft, Stickstoff, anderen Inertgasen oder Gemischen davon thermisch aktiviert wird.

2. Verfahren nach Anspruch 1, **dadurch gekennzeichnet, dass** der calcinierte zeolithische Katalysator maximal 6 Stunden vor Beginn der Umsetzung thermisch aktiviert wird.

3. Verfahren nach Anspruch 1 oder 2, **dadurch gekennzeichnet, dass** die thermische Aktivierung im Aminierungsreaktor durchgeführt wird.

4. Verfahren nach einem der Ansprüche 1 bis 3, **dadurch gekennzeichnet, dass** der calcinierte zeolithische Katalysator vor der thermischen Aktivierung nicht durch eine Hydroaminierung deaktiviert wurde.

5. Verfahren nach einem der Ansprüche 1 bis 4, **dadurch gekennzeichnet, dass** der zeolithische Katalysator ausgewählt ist aus Faujasiten, Erionit, Chabazit, Mordenit, Offretit, Clinoptiolith, Pentasilen, Beta-Zeolithen oder borhaltigen, galliumhaltigen oder titanhaltigen Formen davon sowie Gemischen davon.

6. Verfahren zur Erhöhung der Hydroaminierungsaktivität von calcinierten zeolithischen Katalysatoren, die nicht durch eine Hydroaminierung deaktiviert wurden, durch thermische Behandlung der calcinierten zeolithischen Katalysatoren bei Temperaturen im Bereich von 100 bis 550°C in einem Gasstrom aus Luft, Stickstoff, anderen Inertgasen oder Gemischen davon.

7. Verfahren nach Anspruch 6, **dadurch gekennzeichnet, dass** die thermische Behandlung maximal 24 Stunden vor Beginn einer Hydroaminierung, in der der zeolithische Katalysator eingesetzt wird, durchgeführt wird.

8. Verfahren nach einem der Ansprüche 6 oder 7, **dadurch gekennzeichnet, dass** der calcinierte zeolithische Katalysator vor der thermischen Aktivierung nicht in einer Hydroaminierung eingesetzt wurde.

9. Katalysator, erhältlich nach einem Verfahren gemäß einem der Ansprüche 6 oder 7.
